(19) Europäisches Patentamt
European Patent Office
Office européen des brevets

(11) **EP 2 129 423 B1**

(12) **EUROPÄISCHE PATENTSCHRIFT**

(45) Veröffentlichungstag und Bekanntmachung des Hinweises auf die Patenterteilung:
**04.08.2010 Patentblatt 2010/31**

(21) Anmeldenummer: **08716870.4**

(22) Anmeldetag: **15.02.2008**

(51) Int Cl.:
*A61M 15/00* (2006.01)      *B05B 17/06* (2006.01)
*B67D 7/02* (2010.01)      *B05B 11/02* (2006.01)
*B05B 11/04* (2006.01)

(86) Internationale Anmeldenummer:
**PCT/EP2008/051863**

(87) Internationale Veröffentlichungsnummer:
**WO 2008/113651 (25.09.2008 Gazette 2008/39)**

(54) **AEROSOLGENERATOR**

AEROSOL GENERATOR

GÉNÉRATEUR D'AÉROSOL

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **16.03.2007 DE 102007012804**

(43) Veröffentlichungstag der Anmeldung:
**09.12.2009 Patentblatt 2009/50**

(73) Patentinhaber: **PARI Pharma GmbH**
**82319 Starnberg (DE)**

(72) Erfinder:
• **GALLEM, Thomas**
**81371 München (DE)**
• **HETZER, Uwe**
**81369 München (DE)**
• **HOLZMANN, Philipp**
**85051 Ingolstadt (DE)**

(74) Vertreter: **HOFFMANN EITLE**
**Patent- und Rechtsanwälte**
**Arabellastrasse 4**
**81925 München (DE)**

(56) Entgegenhaltungen:
**EP-A- 1 205 199      WO-A-92/11050**
**DE-A1- 10 102 846      GB-A- 2 291 606**

**Beschreibung**

Die vorliegende Erfindung betrifft einen Aerosolgenerator, insbesondere zur Anwendung in Aerosoltherapiegeräten, wie er im Oberbegriff des Patentanspruchs 1 definiert ist.

Ein derartiger Aerosolgenerator ist z. B. aus der DE 101 02 846 A1 bekannt. Derartige Aerosolgeneratoren besitzen ein Flüssigkeitsreservoir, in dem die zur Zerstäubung vorgesehene Flüssigkeit bevorratet wird. Diese Flüssigkeit wird einem Aerosolerzeuger zugeführt, bei dem es sich um eine Schwingmembran handeln kann. Die auf der einen Seite der Schwingmembran anstehende Flüssigkeit wird dabei durch Öffnungen in der Schwingmembran hindurch transportiert und liegt auf der anderen Seite der Schwingmembran als zu verabreichendes Aerosol vor.

Bereits aus der WO 97/29851 ist es bekannt, dass Aerosolgeneratoren dieser Art besonders effektiv arbeiten, wenn die Flüssigkeit dem Aerosolerzeuger bei einem Druck zugeführt wird, der etwas unterhalb des Umgebungsdrucks in dem Bereich liegt, in den die von der Membran erzeugten Tröpfchen des Aerosols abgegeben werden.

Ferner offenbart die EP 1 205 199 A1 einen Aerosolgenerator mit den Merkmalen des Oberbegriffs von Anspruch 1. Auch ist eine Membran vorgesehen, die eine erste Kammer von einer zweiten Kammer unterteilt, wobei in der ersten Kammer das Flüssigkeitsreservoir angeordnet ist. Die Membran ist durch Zug dehnbar, um das Volumen in der zweiten Kammer zu vergrößern und dadurch einen vorbestimmten Startunterdruck zu erzeugen. In der ersten Kammer herrscht zu diesem Zeitpunkt Umgebungsdruck. Ferner ist die Membran so ausgestaltet, dass sich die Membran unter dem vorgegebenen Unterdruck nicht deformiert.

Die oben erwähnte DE 101 02 846 A1 schlägt zu diesem Zweck eine Dichtung vor, die das offene Ende eines Flüssigkeitsreservoirs gasdicht verschließt und die mit einem Zugelement in Verbindung steht. Durch eine Bewegung des Zugelements wird eine Bewegung zumindest eines Abschnitts der Dichtung bewirkt und dadurch in dem Flüssigkeitsreservoir ein Unterdruck erzeugt, in dem das Volumen des Flüssigkeitsreservoirs bei gleichbleibender Temperatur vergrößert wird (Gesetz nach Boyle-Mariotte).

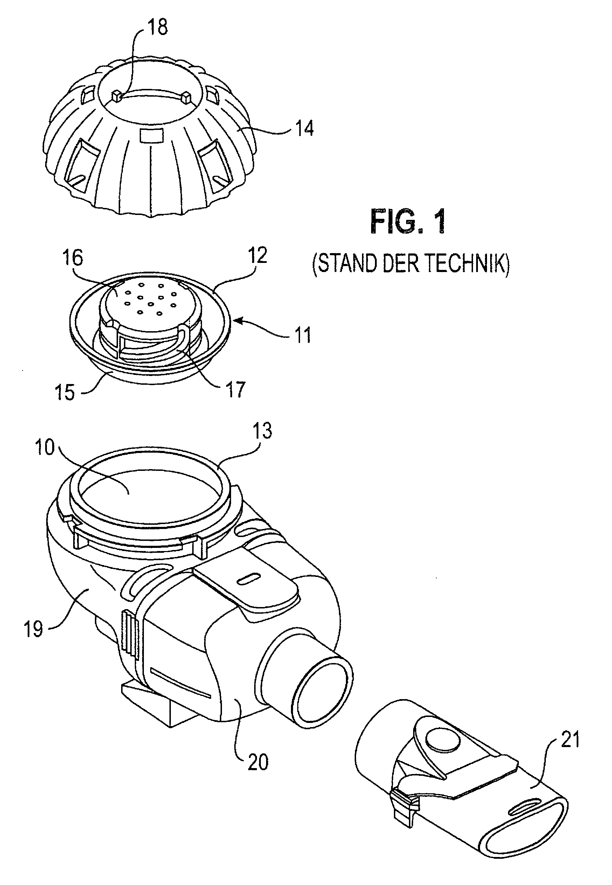

Der in der DE 101 02 846 A1 beschriebene Aerosolgenerator gemäß dem Oberbegriff des Anspruchs 1 ist in leicht abgewandelter Form über die Anmelderin erhältlich. Diese leicht abgewandelte Form ist in den Fig. 1-4 dargestellt.

Fig. 1 zeigt einen Aerosolgenerator mit einem in der Figur nicht dargestellten Aerosolerzeuger. Des Weiteren ist ein Flüssigkeitsreservoir 10 vorgesehen, das einerseits mit dem Aerosolerzeuger in Verbindung steht, so dass die Flüssigkeit an dem Aerosolerzeuger, die im vorliegenden Fall als Schwingmembran ausgebildet ist, ansteht und das andererseits ein offenes Ende aufweist, über welches das Einfüllen der Flüssigkeit erfolgen kann.

Des Weiteren ist eine Dichtung 11 vorgesehen, die dieses offene Ende des Flüssigkeitsreservoirs 10 gasdicht verschließen soll. Dazu ist die Dichtung mit einem umlaufenden Dichtungskragen versehen, der zwischen einer umlaufenden Kante 13 des Flüssigkeitsreservoirs 10 und einer nicht dargestellten umlaufenden Kante des Zugelements, das in Form eines Deckels 14 ausgebildet ist, sandwichartig eingeklemmt wird. Darüber hinaus erstreckt sich von dem Dichtungskragen 12 ein topfförmiger Abschnitt 15, der über das offene Ende des Flüssigkeitsreservoirs zumindest teilweise in diesen hineinragt. Zusätzlich zu dem Dichtungskragen 12 und dem topfförmigen Abschnitt 15, ist die Dichtung 11 mit einem zylindrischen starren Mittelteil 16 versehen, der in seiner Außenumfangsfläche mehrere gewindeartige Führungsbahnen 17 aufweist.

Wie es bereits zuvor erwähnt wurde ist das Zugelement durch einen Deckel 14 gebildet. Der Deckel 14 weist mittig entsprechend dem zylindrischen Mittelteil 16 der Dichtung 11 eine zylindrische Aussparung auf. Auf der Innenumfangsfläche dieser Aussparung sind Vorsprünge 18 angeordnet, die in die Führungsbahnen 17 der Dichtung 11 eingreifen.

Des Weiteren umfasst der dargestellte Aerosolgenerator ein Gehäuse bestehend aus einem das Flüssigkeitsreservoir bildenden Medikamentenbehälter 19 und einer Verneblerkammer 20. An Letztere ist ein Mundstück 21 vorgesehen.

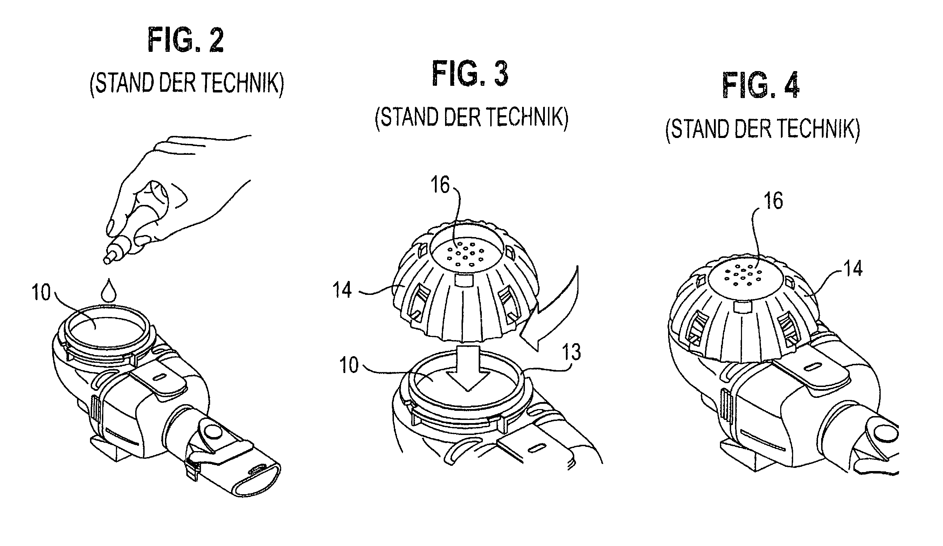

Im Folgenden wird unter Bezugnahme auf die Fig. 2-4 die Funktionsweise dieses bekannten Aerosolgenerators hinsichtlich der Erzeugung eines Unterdrucks erläutert.

Zunächst wird die Flüssigkeit (z. B. ein Medikament) in den Flüssigkeitsbehälter 10 eingefüllt (siehe Fig. 2). Im Anschluss werden die Dichtung 11 und der Deckel 14 zusammengefügt, indem die Vorsprünge 18 in die Führungsbahnen 17 eingeführt werden und in diese eingreifen. Danach wird die Kombination aus Deckel 14 und Dichtung 11 auf die Kante 13 des Flüssigkeitsreservoirs 10 aufgesetzt. Unter leichtem Druck wird der Deckel 14 gedreht (Fig. 3). Durch den Druck wird der Dichtungskragen 12 der Dichtung 11 zwischen der Kante 13 und einer entsprechenden Gegenkante des Deckels 14 sandwichartig eingeklemmt, so dass sich die Dichtung 11 bei der Rotation des Deckels 14 nicht mitdrehen kann. Weiter wird durch den Eingriff der Vorsprünge 18 in den Führungsbahnen 17, welche gewindeartige ausgestaltet sind, die Drehung des Deckels 14 und das Klemmen des Dichtungskragens 12 die Rotation in eine translatorische Bewegung der Dichtung 11 (topfförmiger Abschnitt 15 und Mittelteil 16) in Achsrichtung des zylindrischen Mittelteils 16 umgewandelt. Mit anderen Worten bewegt sich der zylindrische und starre Mittelteil 16 der Dichtung 11 nach oben und zieht daher den in das Flüssigkeitsreservoir 10 ragenden topfförmigen Abschnitt 15 der Dichtung mit nach oben. Dadurch wird das Volumen des Flüssigkeitsreservoirs 10 vergrößert. Da die Dichtung 11 das Flüssigkeitsreservoir 10 jedoch gasdicht verschließt, kann keine Luft nachströmen. Nach Boyle-Mariotte gilt bei konstanter Temperatur:

$$p \times V_{Luft} = konstant$$

Durch die Bewegung der Dichtung 11 wird das Volumen V im Reservoir vergrößert. Damit obige Formel erfüllt ist, muss daher der Druck abnehmen. Es wird ein Unterdruck erzeugt.

Nun ergibt sich die Problematik, dass im Laufe der Vernebelung der Flüssigkeitsspiegel sinkt und dadurch das Luftvolumen $V_{Luft}$ im Reservoir weiter zunimmt. Dementsprechend steigt den obigen Ausführungen zur Folge der Unterdruck im Reservoir mit zunehmend abnehmendem Flüssigkeitsspiegel weiter an. Für spezielle Medikamente (z. B. oberflächenaktive Stoffe (Surfactants) oder andere schäumende Substanzen) kann es jedoch vorteilhaft sein, die Druckbedingungen im Flüssigkeitsreservoir, d. h. den Unterdruck, über die gesamte Vernebelungszeit konstant zu halten. Dies ist insbesondere auch für Anwendungen interessant, bei denen sich die Outputrate (auch als Ausbringungsrate oder Aerosolproduktionsrate bezeichnet) über die Dauer der Vernebelung nicht verändern soll, weil der Unterdruck ein entscheidender Faktor für die Tröpfchengröße und die Cutputrate und damit für die therapeutische Anwendung und Wirksamkeit ist.

Dementsprechend besteht die Aufgabe der vorliegenden Erfindung darin einen Eingangs erwähnten Aerosolgenerator derart weiterzubilden, dass auch im Laufe der Vernebelung und den damit verbundenen abnehmenden Flüssigkeitsspiegel, der Unterdruck im Flüssigkeitsreservoir im Wesentlichen konstant gehalten werden kann.

Diese Aufgabe wird erfindungsgemäß durch einen Aerosolgenerator mit den Merkmalen des Patentanspruchs 1 gelöst und insbesondere dadurch, dass die Dichtung eine Regulierungsmembran aufweist, um den Unterdruck in dem Flüssigkeitsreservoir im Wesentlichen konstant zu halten. Mit anderen Worten ist eine Membran vorgesehen, die mit abnehmendem Flüssigkeitsspiegel das Gesamtluftvolumen des Flüssigkeitsreservoirs im gleichen Maße verringert wie Flüssigkeitsvolumen entnommen wird. Dadurch bleibt das Luftvolumen im Flüssigkeitsreservoir gleich und damit kann auch der Unterdruck konstant gehalten werden. Ein weiterer wesentlicher und wichtiger Vorteil der vorliegenden Erfindung liegt darin, dass durch bloße Umgestaltung der Membrankonfiguration unterschiedliche Unterdruckbereiche eingestellt werden können. D. h. über den Durchmesser, die Stärke und das Material etc. der Membran können ohne Veränderung des Zugelements bzw. der Interaktion zwischen dem Zugelement und der Dichtung (Mittelteil) unterschiedliche Unterdruckbereiche eingestellt werden. Bezüglich des in den Fig. 1-4 beschriebenen Aerosolgenerators bedeutet das, dass für unterschiedliche Druckbereiche ausschließlich die erfindungsgemäße Dichtung 11 ausgetauscht und mit einer entsprechenden anderen Membrankonfiguration versehen werden muss. Der Deckel und die übrigen angrenzenden Bauteile können unverändert bleiben. Auch die Mimik, d. h. die Vorsprünge 18 und die Führungsbahnen 17 können unverändert bleiben. Dies hat unter anderem produktionstechnische Vorteile, weil durch bloßen Austausch eines Elements eine Anpassung des Verneblers an spezielle Anwendungsbereiche möglich ist.

Vorteilhafterweise weist auch die Dichtung der vorliegenden Erfindung einen umlaufenden Dichtungskragen auf, von dem sich ein topfförmiger Abschnitt über das offene Ende zumindest teilweise in das Flüssigkeitsreservoir erstreckt. Durch diese Ausgestaltung ist der Boden bzw. unterer Abschnitt des topfförmigen Abschnitts ähnlich eines Kolbens in dem Flüssigkeitsreservoir angeordnet. Durch Bewegen dieses Kolbens von dem Aerosolerzeuger weg, wird ähnlich wie bei einer Spritze damit auf einfachste Art und Weise ein Unterdruck erzeugt.

Darüber hinaus ist die Dichtung vorzugsweise mit einem zylindrischen starren Mittelteil ausgestattet. Dieser ist jedoch im Gegensatz zum Stand der Technik hohlzylindrisch ausgestaltet und weist wenigstens auf seiner dem Flüssigkeitsbehälter zugewandten Seite ein offenes Ende auf. Die Regulierungsmembran ist dabei derart angeordnet, dass sie die durch das offene Ende gebildete Öffnung verschließt bzw. überbrückt. An dieser Stelle sei angemerkt, dass der Begriff "hohlzylindrisch" auch andere als kreisrunde Querschnittsformen beinhaltet, wie beispielsweise vier- bzw. rechteckige, mehreckige und ovale Querschnittsformen. Auch kann die Querschnittsform am Innenumfang des Hohlzylinders anders sein als an dessen Außenumfang.

Bei einer Ausführungsform der vorliegenden Erfindung erstreckt sich der Boden des topfförmigen Abschnitts, und damit der topfförmige Abschnitt, über die Öffnung. Zumindest in einem Bereich, in dem der topfförmige Abschnitt die Öffnung überbrückt, ist der topfförmige Abschnitt als Regulierungsmembran ausgebildet, d. h. dieser Abschnitt ist bezüglich Durchmesser, Stärke und Material derart ausgebildet, dass die Regulierung bewirken kann. Auch ist es denkbar, dass nur ein Teilbereich des genannten Bereichs des topfförmigen Abschnitts die Regulierungsmembran bildet.

Alternativ ist der topfförmige Abschnitt zumindest in einem Teilbereich der Öffnung ausgespart und die Regulierungsmembran ist an einer in Achsrichtung des Hohlzylinders beliebigen Stelle in dem Hohlzylinder angeordnet. Dies ermöglicht eine Ausgestaltung der Membran bezüglich Durchmesser, Stärke und Material im Wesentlichen unabhängig von dem topfförmigen und anderen Abschnitten der Dichtung.

Jedoch ist es bevorzugt, dass der Dichtungskragen, der topfförmige Abschnitt und die Regulierungsmembran unabhängig davon, ob sie ein Teil des topfförmigen Abschnitts ist oder getrennt davon ausgebildet ist aus dem gleichen Material gebildet sind. Dadurch wird die Möglichkeit geschaffen, die Dichtung in einem Zweikomponentenspritzgießverfahren als integrales Bauteil auszubilden. Eine Komponente bildet der Mittelteil aus z. B. hartem Kunststoff und die andere Komponente wird durch den weichelastischen Kunststoff der übrigen Dichtungskomponenten vorgegeben.

Um bei dem bestehenden Therapiegerät einen Unterdruck von ungefähr 150 mbar zu erzeugen, weist die Regulierungsmembran vorzugsweise eine Härte von ungefähr 25 Shore A auf. Um ein Unterdruck von z. B. 250 mbar zu erzeugen, kann eine Härte von 60 oder 70 Shore A geeignet sein.

Darüber hinaus weist die Regulierungsmembran bevorzugt eine Stärke zwischen 0,3 und 0,8 mm auf.

Weitere Merkmale und Vorteile der vorliegenden Erfindung ergeben sich aus der folgenden Beschreibung einer bevorzugten Ausführungsform, die unter Bezugnahme auf die begleitenden Zeichnungen erfolgt.

In den Zeichnungen zeigen:

Fig. 1-4 zeigt einen Aerosolgenerator des Standes der Technik, sowie den Funktionsablauf zum Erzeugen des Unterdrucks bei einem solchen Aerosolgenerator.

Fig. 5 eine Dichtung mit einer Regulierungsmembran gemäß der vorliegenden Erfindung, die die Dichtung in dem Aerosolgenerator gemäß den Fig. 1-4 ersetzt.

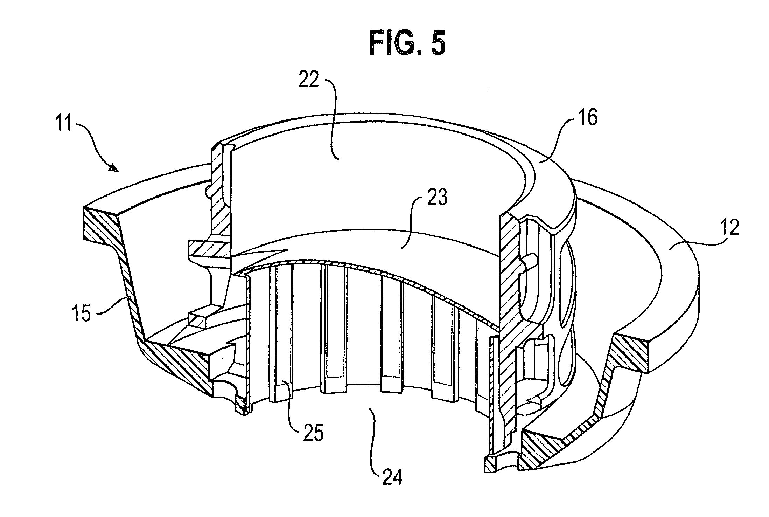

Die vorliegende Erfindung betrifft einen Aerosolgenerator, wie er Eingangs in Bezug auf die Fig. 1-4 beschrieben wurde. Einzig ist die Dichtung 11 anders ausgestaltet. Bezüglich der anderen Merkmalen des erfindungsgemäßen Aerosolgenerators wird, um Wiederholungen zu vermeiden, daher auf die vorherigen Ausführungen bezüglich der Fig. 1-4 Bezug genommen. Gleiche oder entsprechende Teile der Dichtung 11 in Fig. 5 sind mit den gleichen Bezugszeichen gekennzeichnet wie die Merkmale der Dichtung in Fig. 1.

Die in Fig. 5 dargestellte erfindungsgemäße Dichtung 11 umfasst ebenfalls einen Dichtungskragen 12 und einen sich von dem Dichtungskragen erstreckenden topfförmigen Abschnitt 15. Des Weiteren ist auch ein starrer Mittelteil 16 vorgesehen, wobei sich der topfförmige Abschnitt 15 sowie der Dichtungskragen 12 um dessen Umfang erstrecken. Auch die Führungsbahnen 17 für den Eingriff mit den Vorsprüngen 18 sind vorgesehen.

Die Dichtung 11 der vorliegenden Erfindung unterscheidet sich dadurch, dass der starre Mittelteil 16 hohlzylindrisch ausgebildet ist. D. h. der starre Mittelabschnitt 16 weist eine Durchbrechung 22 auf, die sich bei der dargestellten Ausführungsform vollständig durch den zylindrischen Mittelteil erstreckt, so dass dieser zwei offene Enden aufweist. In dem durch diese Durchbrechung 22 geformten Hohlzylinder ist etwa mittig die Regulierungsmembran 23 angeordnet. Der topfförmige Abschnitt 15 ist fluchtend mit der Durchbrechung 22 ebenfalls ausgespart. D. h. es ergibt sich im Bereich des Endes des starren Mittelteils 16, das dem Flüssigkeitsreservoir zugewandt ist, eine Öffnung 24, die mit dem Flüssigkeitsreservoir in Verbindung steht. Diese Öffnung wird bei der dargestellten Ausführungsform durch die Regulierungsmembran 23 verschlossen.

Wie es Fig. 5 weiter zu entnehmen ist, sind die Regulierungsmembran 23, der topfförmige Abschnitt 15 und der Dichtungskragen 12 aus dem gleichen Material gebildet. Bei diesem Material kann es sich um einen thermoplastischen Kunststoff, vorzugsweise einen thermoplastischen Elastomer handeln. Die Stärke der Dichtung im topfförmigen Abschnitt 15 beträgt ca. 0,3 bis 0,8 mm. Diese Stärke entspricht auch der bevorzugten Stärke der Regulierungsmembran 23. Daher ist es denkbar die Regulierungsmembran auch als Fortsatz des topfförmigen Abschnitts 15 auszugestalten, der sich über die Öffnung erstreckt bzw. diese überbrückt. D. h. der topfförmige Abschnitt 15 wäre nicht - wie zuvor erwähnt - ausgespart.

Der starre Mittelteil 16 besteht vorzugsweise ebenfalls aus einem gießfähigen aber harten Kunststoff, so dass die Dichtung 11 insgesamt als integrales Zweikomponentenspritzgussbauteil ausgestaltet werden kann. Zu diesem Zweck ist zumindest ein Teilabschnitt des Innenumfangs des hohlzylindrischen Mittelteils 16 mit abwechselnd Rippen und Kanälen ausgestattet, wobei sich im Spritzgießverfahren die zwischen den Rippen 25 angeordneten Kanäle ebenfalls mit Kunststoff füllen, wodurch eine ortsgenaue Festlegung der Regulierungsmembran 23 geschaffen wird.

Die obige Beschreibung bezieht sich ausschließlich auf eine bevorzugte Ausführungsform der vorliegenden Erfindung. Es sind jedoch auch andere Ausgestaltungen denkbar. So muss der Mittelabschnitt 16 nicht zwingend zwei offene Enden aufweisen. Es reicht, wenn im Bereich der Öffnung 24 ein offenes Ende vorgesehen ist, so dass Volumen des Flüssigkeitsreservoirs von der Regulierungsmembran 23 begrenzt wird. Auch ist nicht zwingend die Ausgestaltung der Führungsbahnen 17 in gewindeähnlicher Form notwendig. Andere Ausgestaltungen mittels derer die Dichtung so bewegt werden kann, dass sich das Volumen im Flüssigkeitsreservoir 10 ändert, sind denkbar. Dementsprechend ist die oben beschriebene Ausführungsform nicht als die vorliegende Erfindung begrenzend anzusehen. Vielmehr ergibt sich der Schutzumfang aus den folgenden Patentansprüchen.

**Patentansprüche**

1. Aerosolgenerator, insbesondere für Aerosoltherapiegeräte, mit

    - einem Aerosolerzeuger, um aus einer ihm zugeführten Flüssigkeit ein Aerosol zu erzeugen,
- einem Flüssigkeitsreservoir (10) für die Aufnahme der Flüssigkeit, das zur Zuführung der Flüssigkeit einerseits mit dem Aerosolerzeuger in Verbindung steht und andererseits ein offenes Ende aufweist,
- einer Dichtung (11), die das offene Ende des Flüssigkeitsreservoirs gasdicht verschließt, und
- einem Zugelement (14), das derart mit der Dichtung (11) in Verbindung steht, dass eine Bewegung des Zugelements eine Bewegung zumindest eines Abschnitts der Dichtung bewirkt und dadurch in dem Flüssigkeitsreservoir ein Unterdruck erzeugt wird, **dadurch gekennzeichnet, dass**

die Dichtung eine Regulierungsmembran (23) aufweist, die mit abnehmendem Flüssigkeitsspiegel der Flüssigkeit in dem Flüssigkeitsreservoir ein Gesamtluftvolumen in dem Flüssigkeitsreservoir derart verringert, dass der Unterdruck in dem Flüssigkeitsreservoir im Wesentlichen konstant bleibt.

**2.** Aerosolgenerator nach Anspruch 1, **dadurch gekennzeichnet, dass** die Dichtung (11) einen umlaufenden. Dichtungskragen (12) aufweist, von dem sich ein topfförmiger Abschnitt (15) über das offene Ende zumindest teilweise in das Flüssigkeitsreservoir erstreckt.

**3.** Aerosolgenerator nach Anspruch 2, **dadurch gekennzeichnet, dass** die Dichtung (11) ferner einen hohlzylindrischen, starren Mittelteil (16) aufweist, der wenigstens auf seiner dem Flüssigkeitsreservoir (10) zugewandten Seite ein offenes Ende aufweist, wobei die Regulierungsmembran (23) derart angeordnet ist, dass sie die durch das offene Ende gebildete Öffnung (24) verschließt.

**4.** Aerosolgenerator nach Anspruch 3, **dadurch gekennzeichnet, dass** sich der topfförmige Abschnitt (15) über die Öffnung (24) erstreckt und in seinem die Öffnung verschließenden Teil als Regulierungsmembran (23) ausgebildet ist.

**5.** Aerosolgenerator nach Anspruch 3, **dadurch gekennzeichnet, dass** der topfförmige Abschnitt (15) zumindest in einem Teilbereich der Öffnung (24) ausgespart ist und die Regulierungsmembran an einer in Achsrichtung des Hohlzylinders beliebigen Stelle in dem Hohlzylinder angeordnet ist.

**6.** Aerosolgenerator nach einem der Ansprüche 2 bis 5, **dadurch gekennzeichnet, dass** der Dichtungskragen (12), der topfförmige Abschnitt (15) und die Regulierungsmembran (23) aus dem gleichen Material gebildet sind.

**7.** Aerosolgenerator nach einem der Ansprüche 3 bis 6, **dadurch gekennzeichnet, dass** die Dichtung (11) ein integrales Bauteil, vorzugsweise ein 2-Komponenten Spritzgussteil, aus einem weichelastischen und einem harten Kunststoff, ist.

**8.** Aerosolgenerator nach einem der vorstehenden Ansprüche, **dadurch gekennzeichnet, dass** die Regulierungsmembran (23) eine Härte zwischen 25 Shore A und 70 Shore A aufweist.

**9.** Aerosolgenerator nach einem der vorstehenden Ansprüche, **dadurch gekennzeichnet, dass** die Regulierungsmembran (23) eine Stärke zwischen 0,3 und 0,8 mm aufweist.

## Claims

**1.** Aerosol generator, in particular for aerosol treatment devices, having

- an aerosol generating means for generating an aerosol from a liquid delivered to it,
- a liquid reservoir (10) for holding the liquid, which for delivery of the liquid on one side is connected to the aerosol generating means and on the other side has an open end,
- a seal (11) which closes the open end of the liquid reservoir gastightly, and
- a pull element (14) which is connected to the seal (11) in such a way that a movement of the pull element causes a movement of at least one section of the seal and so a partial pressure is generated in the liquid reservoir, **characterised in that**

the seal has a regulating diaphragm (23) which, with a decrease in the liquid level of liquid in the liquid reservoir, reduces a total air volume in the liquid reservoir in such a way that the partial pressure in the liquid reservoir remains substantially constant.

**2.** Aerosol generator according to claim 1, **characterised in that** the seal (11) has a peripheral sealing collar (12) from which a cup-shaped section (15) extends over the open end at least partially into the liquid reservoir.

**3.** Aerosol generator according to claim 2, **characterised in that** the seal (11) further has a hollow cylindrical rigid centre portion (16) which has an open end at least on its side facing towards the liquid reservoir (10), the regulating diaphragm (23) being arranged so as to close the opening (24) formed by the open end.

**4.** Aerosol generator according to claim 3, **character-**

**ised in that** the cup-shaped section (15) extends over the opening (24) and is designed as a regulating diaphragm (23) in its portion that closes the opening.

5. Aerosol generator according to claim 3, **characterised in that** the cup-shaped section (15) is recessed at least in part of the opening (24) and the regulating diaphragm is arranged at any location in the hollow cylinder in the axial direction of the hollow cylinder.

6. Aerosol generator according to any of claims 2 to 5, **characterised in that** the sealing collar (12), the cup-shaped section (15) and the regulating diaphragm (23) are made of the same material.

7. Aerosol generator according to any of claims 3 to 6, **characterised in that** the seal (11) is an integral component, preferably a two-component injection moulding, made of a flexible and a hard plastic.

8. Aerosol generator according to any of the preceding claims, **characterised in that** the regulating diaphragm (23) has a hardness of between 25 Shore A and 70 Shore A.

9. Aerosol generator according to any of the preceding claims, **characterised in that** the regulating diaphragm (23) has a thickness of between 0.3 and 0.8 mm.

## Revendications

1. Générateur d'aérosol, en particulier pour des appareils de thérapie par aérosol, avec

   - un producteur d'aérosol, pour produire un aérosol à partir d'un liquide lui étant amené,
   - un réservoir à liquide (10), pour recevoir le liquide, d'une part, relié au producteur d'aérosol pour l'amenée de liquide et, d'autre part, présentant une extrémité ouverte,
   - un joint d'étanchéité (11), obturant de manière étanche aux gaz l'extrémité ouverte du réservoir à liquide, et
   - un élément de traction (14), relié au joint d'étanchéité (11) de manière qu'un déplacement de l'élément de traction provoque un déplacement au moins d'un tronçon du joint d'étanchéité et que, de ce fait, une dépression soit produite dans le réservoir à liquide,

   **caractérisé en ce que**
   le joint d'étanchéité présent une membrane de régulation (23) qui, lorsque le niveau de liquide diminue dans le réservoir à liquide, diminue un volume d'air global dans le réservoir à liquide, de manière que la dépression dans le réservoir à liquide reste sensiblement constante.

2. Générateur d'aérosol selon la revendication 1, **caractérisé en ce que** le joint d'étanchéité (11) présente une collerette d'étanchéité (12) de pourtour, dont un tronçon (15) en forme de pot, au-dessus de l'extrémité ouverte, s'étend au moins partiellement dans le réservoir à liquide.

3. Générateur d'aérosol selon la revendication 2, **caractérisé en ce que** joint d'étanchéité (11) présente en outre une partie médiane (16) rigide, cylindrique creuse, présentant, au moins sur sa face tournée vers le réservoir à liquide (10), une extrémité ouverte, la membrane de régulation (23) étant disposée de manière qu'elle obture l'ouverture (24) formée par l'extrémité ouverte.

4. Générateur d'aérosol selon la revendication 3, **caractérisé en ce que** le tronçon (15) en forme de pot s'étend sur l'ouverture (24) et, dans sa partie obturant l'ouverture, est réalisée sous forme de membrane de régulation (23).

5. Générateur d'aérosol selon la revendication 3, **caractérisé en ce que** le tronçon (15) en forme de pot est évidé, au moins dans une zone partielle de l'ouverture (24), et la membrane de régulation est disposée, à un emplacement quelconque en direction axiale du cylindre creux, à l'intérieur du cylindre creux.

6. Générateur d'aérosol selon l'une des revendications 2 à 5, **caractérisé en ce que** la collerette d'étanchéité (12), le tronçon (15) en forme de pot et la membrane de régulation (23) sont formés du même matériau.

7. Générateur d'aérosol selon l'une des revendications 3 à 6, **caractérisé en ce que** le joint d'étanchéité (11) est un composant monobloc, de préférence une pièce moulée par injection à 2 composants, formée d'une matière synthétique élastiquement souple et d'une autre dure.

8. Générateur d'aérosol selon l'une des revendications précédentes, **caractérisé en ce que** la membrane de régulation (23) présente une dureté dans la fourchette comprise entre 25 Shore A et 70 Shore A.

9. Générateur d'aérosol selon l'une des revendications précédentes, **caractérisé en ce que** la membrane de régulation (23) présente une épaisseur dans la fourchette comprise entre 0,3 et 0,8 mm.

**FIG. 1**

(STAND DER TECHNIK)

# FIG. 2
### (STAND DER TECHNIK)

# FIG. 3
### (STAND DER TECHNIK)

# FIG. 4
### (STAND DER TECHNIK)

EP 2 129 423 B1

# FIG. 5

EP 2 129 423 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10102846 A1
- WO 9729851 A
- EP 1205199 A1